# FASCICULE DE BREVET EUROPEEN

(11) **EP 0 946 579 B1**
(45) Date de publication et mention de la délivrance du brevet: **02.05.2002**
(21) Numéro de dépôt: 97951293.6
(22) Date de dépôt: 10.12.1997
(51) Int. Cl.: C07H 17/08, A61K 31/70

(54) **NOUVEAUX DERIVES DE L'ERYTHROMYCINE, LEUR PROCEDE DE PREPARATION ET LEUR APPLICATION COMME MEDICAMENTS**
ERYTHROMYCINDERIVATE, VERFAHREN ZU IHRER HERSTELLUNG, UND IHRER VERWENDUNG ALS ARZNEIMITTELN
NOVEL ERYTHROMYCIN DERIVATIVES, METHOD FOR PREPARING THEM AND THEIR USE AS MEDICINE

(30) Priorité: 12.12.1996 FR 9615271
(43) Date de publication de la demande: 06.10.1999
(73) Titulaire: Aventis Pharma S.A., 92160 Antony (FR)
(72) Inventeur: AUGER, Jean-Michel, F-93100 Montreuil (FR); AGOURIDAS, Constantin, F-94130 Nogent sur Marne (FR); CHANTOT, Jean-François, F-94130 Nogent sur Marne (FR); DENIS, Alexis, F-75011 Paris (FR)
(86) Numéro de dépôt international: FR9702254
(87) Numéro de publication internationale: WO9825942

(56) Documents cités:
- EP-A- 0 248 279
- EP-A- 0 487 411
- EP-A- 0 596 802
- EP-A- 0 638 585
- EP-A- 0 676 409
- EP-A- 0 680 967
- BAKER W R ET AL: "Modification of macrolide antibiotics. Synthesis of 11-deoxy-11-(carboxyamino)-6-O-methyleryth romycin A 11,12-(cyclic esters) via an intramolecular Michael reaction of O-carbamates with an.alpha.,.beta.-unsaturated ketone" J. ORG. CHEM. (JOCEAH,00223263);88; VOL.53 (10); PP.2340-5, ABBOTT LAB.;PHARM. PROD. DIV.; ABBOTT PARK; 60064; IL; USA (US), XP002026111

## Description

La présente invention concerne de nouveaux dérivés de l'érythromycine, leur procédé de préparation et leur application comme médicaments.

L'invention a pour objet les composés de formule (I) : 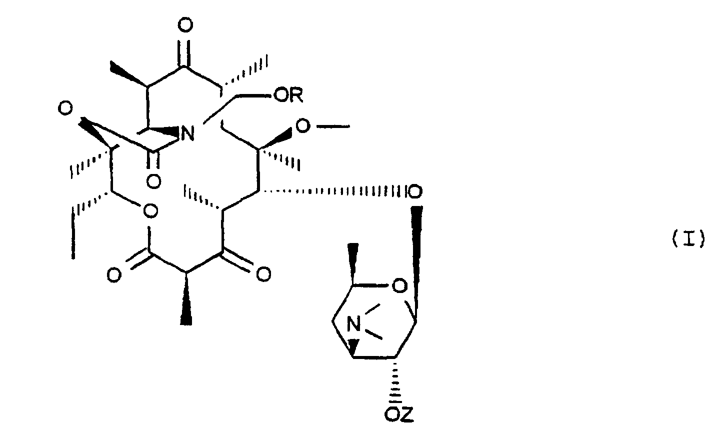 dans lesquels R représente :
ou bien un radical alkyle, alkényle ou alkynyle, linéaire, ramifié ou cyclique renfermant jusqu'à 18 atomes de carbone, éventuellement substitué par un ou plusieurs substituants choisis parmi les atomes d'halogène, les radicaux O-alkyle, O-alkényle ou O-alkynyle, S-alkyle, S-alkényle ou S-alkynyle, linéaires, ramifiés ou cycliques, renfermant jusqu'à 12 atomes de carbone, les radicaux NO₂, les radicaux C≡N, les radicaux : 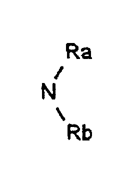 dans lesquels Ra et Rb identiques ou différents représentent un atome d'hydrogène, un radical alkyle linéaire ou ramifié renfermant jusqu'à 4 atomes de carbone, les radicaux : 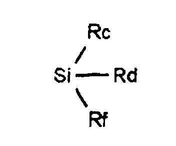 dans lesquels Rc, Rd et Rf identiques ou 'différents, représentent un radical alkyle, linéaire, ramifié ou cyclique renfermant jusqu'à 4 atomes de carbone, éventuellement substitué par un ou plusieurs atomes d'halogène,
ou bien un radical (CH₂)ₙAr dans lequel n représente un nombre entier allant de 0 à 6 et Ar représente un radical aryle ou hétéroaryle, éventuellement substitué par un ou plusieurs substituants indiqués ci-dessus,
Z représente un atome d'hydrogène ou le reste d'un radical acyle, renfermant jusqu'à 12 atomes de carbone, ainsi que leurs sels d'addition avec les acides.

Le radical aryle peut être un radical phényle ou naphtyle.

Le radical aryle peut être également un radical hétérocyclique substitué ou non comme le radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NH₂, NO₂, C≡N, les radicaux alkyle, alkényle ou alkynyle, 0-alkyle, 0-alkényle ou 0-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 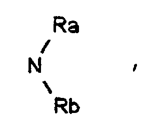 Ra et Rb identiques ou différents, représentant un atome d'hydrogène ou un radical alkyle renfermant jusqu'à 12 atomes de carbone, le radical 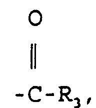 R₃ représentant un radical alkyle renfermant jusqu'à 12 atomes de carbone, ou un radical aryle ou hétéroaryle à 5 ou 6 chaînons comportant un ou plusieurs hétéroatomes, éventuellement substitués par un ou plusieurs des substituants mentionnés ci-dessus.
Comme hétérocycle préféré, on peut citer entre autres 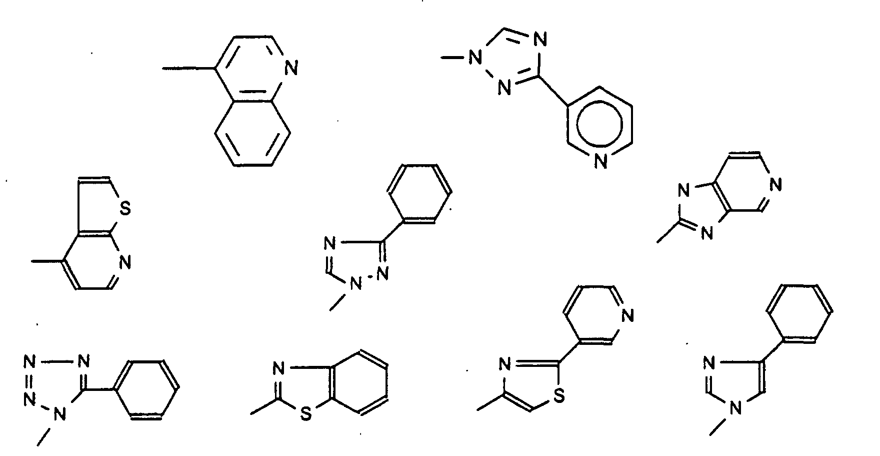 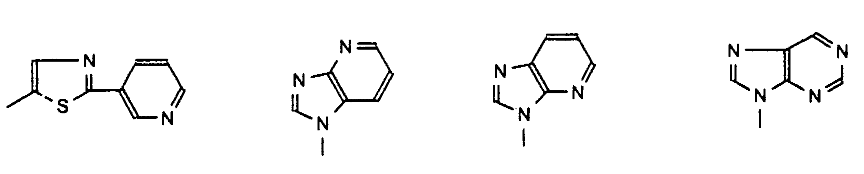 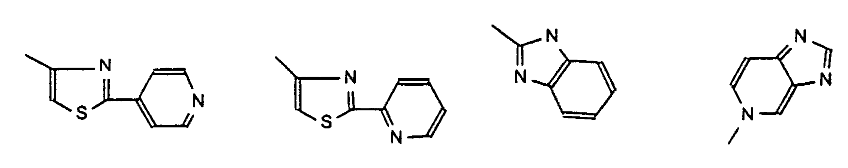 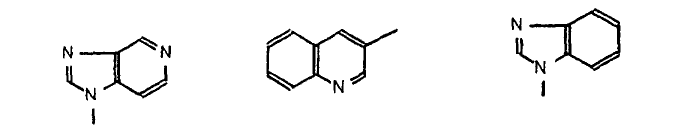 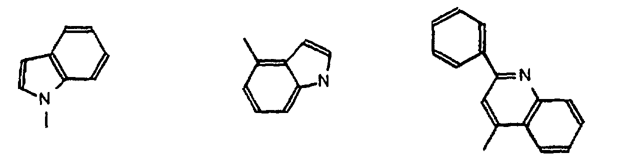 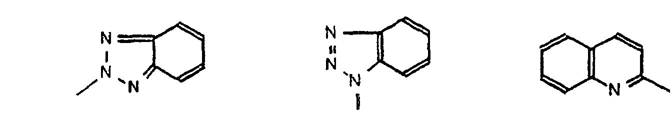 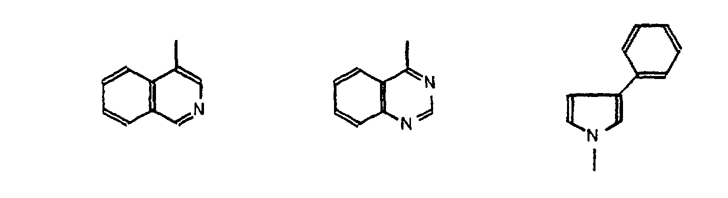 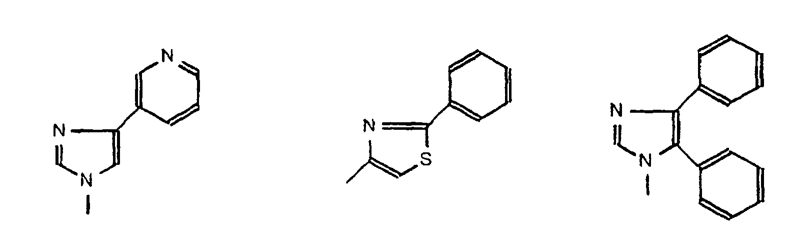 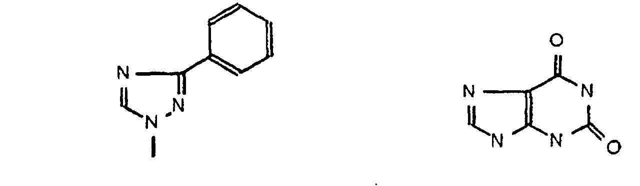 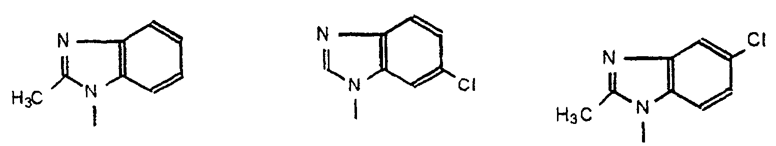 et les radicaux hétérocycliques envisagés dans les demandes de brevets européens 487411, 596802, 676409 et 680967. Ces radicaux hétérocycliques préférés pouvant être substitués par un ou plusieurs groupements fonctionnels.
Hal représente de préférence un atome de fluor, de chlore ou de brome.

Parmi les sels d'addition avec les acides, on peut citer les sels formés avec les acides acétique, propionique, trifluoroacétique, malique, tartrique, méthanesulfonique, benzènesulfonique, p-toluènesulfonique, et spécialement les acides stéariques, éthylsuccinique ou laurylsulfonique.

L'invention a notamment pour objet les composés de formule (I) dans lesquels Z représente un atome d'hydrogène, les composés de formule (I) dans lesquels R représente un radical (CH₂)ₙAr dans lesquels n représente un nombre entier allant de 1 à 4 et Ar représente un radical aryle ou hétéroaryle éventuellement substitué, et notamment ceux dans lesquels Ar est un radical phényle éventuellement substitué, ainsi que ceux dans lesquels R représente un radical : 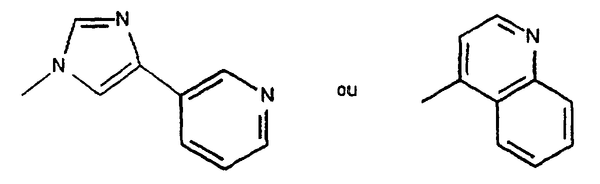 éventuellement substitué.

L'invention a tout particulièrement pour objet les composés dont la préparation est donnée ci-après dans la partie expérimentale et notamment les produits des exemples 2 et 3.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, broncho-pneumonies, suppurations pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples 2 où 3 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 2.

L'invention a également pour objet un procédé de préparation des composés de formule (I), caractérisé en ce que l'on soumet un composé de formule (II) : 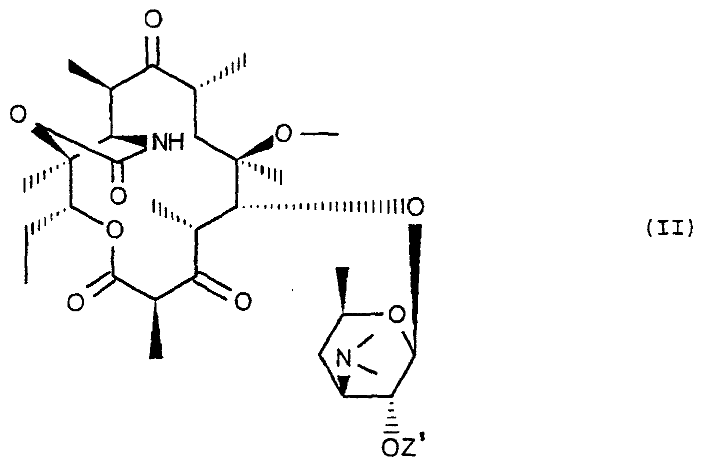 dans lequel Z' représente le reste d'un acide carboxylique renfermant jusqu'à 12 atomes de carbone, à l'action d'un agent de blocage de la fonction cétone en 3 sous forme d'éther ou d'ester d'énol pour obtenir le composé de formule (III) : 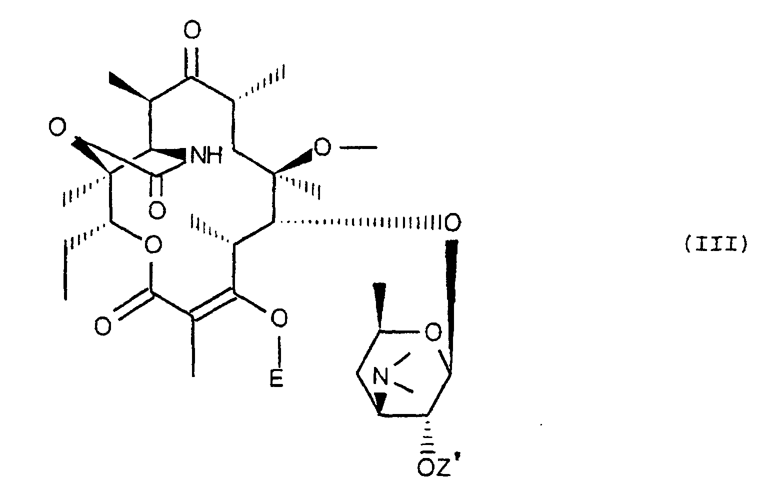 dans lequel E représente le reste d'un éther ou d'un ester d'énol et Z' conserve sa signification précédente, à l'action d'un composé de formule (IV) :

Hal-CH₂OR (IV)

dans laquelle Hal représente un atome d'halogène, pour obtenir le composé de formule (V) correspondant : 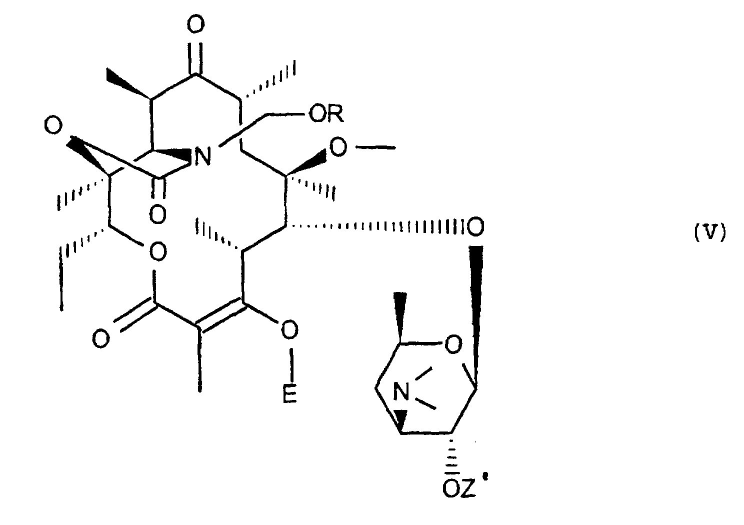 que l'on soumet, si désiré, à l'action d'un agent de libération de la fonction cétone en 3 et/ou à l'action d'un agent de libération de l'hydroxyle en 2', pour obtenir le composé de formule (I) correspondant : 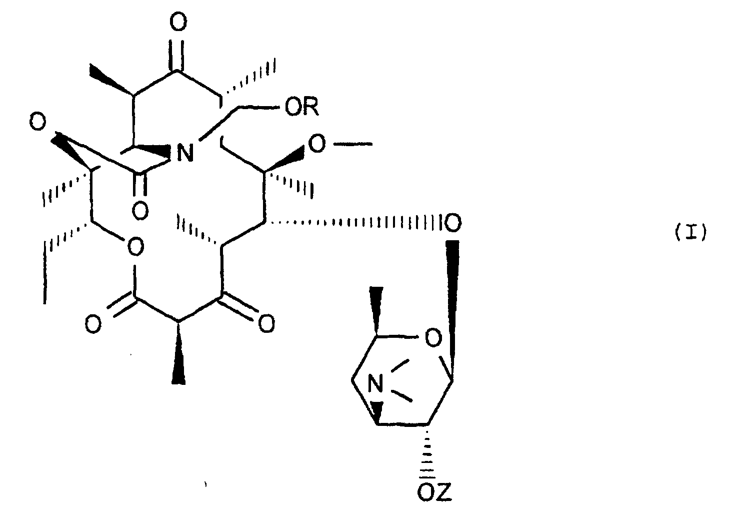 dans lequel R et Z conservent leur signification précédente.

Les produits de formule (II) utilisés comme produits de départ sont des produits connus, qui peuvent être préparés selon le procédé décrit dans EP 487411 ou dans WO 9321199.

Comme agent de blocage de la fonction cétone en 3, sous forme d'éther d'énol, on peut utiliser un hologénométhyléther et notamment un chlorométhéthyléther, comme par exemple le chlorure de MEM, ou chlorure de 2-méthoxy éthoxy méthyle ou encore le chlorure de SEM ou chlorure de 2-(triméthylsilyl) éthoxyméthyle ; on peut également utiliser le benzyloxyméthyléther (BOM) comme agent de blocage sous forme d'ester d'énol.
- Hal représente de préférence un atome de chlore,
- Z' représente de préférence un radical acétyle,
- l'agent de libération de la fonction cétone en 3 ou
- la libération de l'hydroxyle en 2' est effectuée par méthanolyse.

Les composés de formules (III) et (V) mis en oeuvre au cours du procédé de préparation sont nouveaux et sont en eux-mêmes un objet de la présente invention.

Les exemples suivants illustrent l'invention sans toutefois la limiter.

### EXEMPLE 1 : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[(2-méthoxyéthoxy)méthyl]imino]]-érythromycine

### Stade A : 2'-acétate de 2,3-didéhydro-11,12-didéoxy-3-O-de[(2,6-didéoxy-3-C-méthyl-3-O-méthyl-alpha-L-ribo-hexopyranosyl)-6-O-méthyl-11,12-(iminocarbonyloxy) -3-O-[[(2-triméthylsilyl) éthoxy] méthyl]-érythromycine

On agite pendant 10 minutes une solution renfermant 6 ml de DMF, 0,654 g de 2'-acétate de 11,12-didéoxy-3-de[(2,6-didéoxy-3-C-méthyl-alpha-L-ribo-hexopyranosyl) oxy]-6-O-méthyl-3-oxo-11,12-(iminocarbonyloxy)-érythromycine et 0,057 g d'hydrure de sodium à 50 % dans l'huile. On porte le mélange réactionnel à 40°C. On refroidit à -5°C et introduit goutte à goutte 0,177 µl de ClSEM. On dilue dans 5 ml de DMF et amène à pH 7. On ajoute 3 gouttes d'eau, amène à la température ambiante et évapore le DMF. On reprend dans 15 ml d'acétate d'éthyle, lave avec une solution aqueuse d'ammoniaque, extrait à l'acétate d'éthyle, lave à l'eau, sèche, filtre et concentre. On obtient 0,785 g de produit que l'on dissout dans l'éther isopropylique. On amorce la cristallisation, essore, lave et sèche à 70°C. On obtient le produit recherché fondant à 100°C.
RMN CDCl₃ ppm
Structure possible
0,07 (s) : Si(Me)₃ ; 0,87 (t) : CH₃-CH₂ ; ~ 1,03 : CH₂-Si ; 1,10 (d) - 1,12 (d) - 1,14 (d) - 1,24 (d) les CH₃ ; 1,24 (s) - 1,43 (s) : 6 et 12 CH₃ ; 1,95 (s) : 2-Me ; 2,08 (s) : OAc ; 2,27 (s) : N(Me)₂ ; 2,47 (m) : H₈ ; 2,68 (m) : H'₃ ; 2,82 (s) : 6-OMe ; 3,04 (q) : H₁₀ ; 3,31 (dq) : H₄ ; 3,48 (m) : H'₅ ; 3,73 (d,J = 2,5) : H₅ ; 3,80-3,92 : OCH₂ ; 3,89 (s) : H₁₁ ; 4,69 (d) : H₁ ; 4,78 (dd) : H₂ ; 4,99 (d) - 5,04 (d) : OCH₂O ; 5,29 (dd) : H₁₃.

### Stade B : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[(méthoxyméthyl)]imino]]érythromycine

On dissout dans 1,5 ml de DMF, 0,150 g d'un mélange renfermant 0,150 g du produit du stade A.

On refroidit le mélange réactionnel à +10°C et ajoute 14 mg d'hydrure de sodium à 50 % dans l'huile. On refroidit le mélange réactionnel à 0°C et introduit 26 µl de chlorure de MEM en solution dans 0,5 ml de DMF. On agite pendant 25 minutes à 0°C, verse sur la glace, concentre, reprend à l'acétate d'éthyle, lave à l'eau, extrait à l'acétate d'éthyle, sèche, filtre et concentre. On obtient un produit que l'on dilue dans 4 ml de méthanol. On porte la solution au reflux pendant 2 heures, ramène à la température ambiante. On ajoute 1 ml d'une solution 6,5 M d'acide chlorhydrique dans le méthanol. On évapore le méthanol, reprend à l'acétate d'éthyle, lave à l'ammoniaque, dilue, extrait à l'acétate d'éthyle, sèche, filtre et concentre. On obtient 0,11 g de produit qui cristallise. On obtient après purification, le produit recherché brut F = 238∼240°C.
RMN CDCl₃ ppm
0,88 (t) : CH₃-CH₂ ; 1,03 (d) : 10-Me ; 1,16 (d) : 8Me ; 1,25 (d) : 5'Me ; 1,32 (d) : 4Me ; 1,38 (d) : 2Me ; 1,34 et 1,51 : O et 12Me ; ~ 1,57 et 1,38 CH₂ en 14; ~ 1,60 et 1,84 : CH₂ en 7 ; ~ 1,67 et 1,25 : CH₂ en 4' ; 2,27 (s) : N(Me)₂ ; 2,44 (m) : H'₃ ; 2,62 (m) : H₈ ; 2,67 (s) : 6-OMe ; 3,09 (ql) : H₁₀ ; 3,12 (m) : H₄ ; 3,18 (dd) : H'₂ ; 3,36 (s) : OMe ; ~ 3,47 : OH ; 3,86 (q) : H₂ ; 3,87 (s) : H₁₁ ; 4,24 (d) : H'₁ ; 4,93 (d) - 5,27 (d) : NCH₂O ; 5,05 (dd) : H₁₃.

### EXEMPLE 2 : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[[2-[4-(3-pyridinyl)-1H-imidazol-1-yl] éthoxyl méthyl] imino]]-érythromycine

### Stade A : 2'-acétate de 2,3-didéhydro-11,12-didéoxy-3-O-de(2,6-didéoxy-3-C-méthyl-3-O-méthyl-alpha-L-ribo-hexopyranosyl)-6-O-méthyl-12,11-[oxycarbonyl[[(2-bromoéthoxy) méthyl] imino]]-3-O-[[2-(triméthylsilyl) éthoxy] méthyl]-érythromycine

Dans 2 ml de THF, on agite 0,278 g d'hydrure de sodium à 50 % dans l'huile. On refroidit à 0°C et ajoute 510 mg de stade A de l'exemple 1, en solution dans 8 ml de THF. On ramène à la température ambiante et introduit 100 µl de ClCH₂OCH₂CH₂Br en solution dans 4 ml de THF. On ramène à 0°C, verse sur glace, extrait à l'acétate d'éthyle, lave à l'eau salée, sèche, filtre et concentre. On obtient 0,709 g du produit recherché.

### Stade B : 2'-acétate de 2,3-didéhydro-11,12-didéoxy-3-O-de(2,6-didéoxy-3-C-méthyl-3-O-méthyl-alpha-L-ribo-hexopyranosyl)-6-O-méthyl-12,11-[oxycarbony[[[2-[4-(3-pyridinyl)-1H-imidazol-1-yl] éthoxy] méthyl] imino]]-3-O-[[2-(triméthylsilyl) éthoxyl méthyl]-érythromycine

On introduit une solution de 3 ml de DMF et 0,377 g de 4-(3-pyridinyl)-1H-imidazole dans une solution renfermant 2 ml de DMF et 0,162 g d'hydrure de sodium à 50 % dans l'huile. On agite pendant un quart d'heure et introduit 0,709 g du produit du stade A de l'exemple 2 en solution dans 8 ml de DMF. On agite 3 heures à la température ambiante et 15 minutes à 60°C. On verse sur la glace, extrait à l'acétate d'éthyle, lave à l'eau salée puis à l'eau, sèche, filtre et concentre. On obtient 0,644 g de produit.

### Stade C : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[[2-[4-(3-pyridinyl)-1H-imidazol-1-yl] éthoxy] méthyl] imino]]-érythromycine

On agite pendant 48 heures à la température ambiante une solution renfermant 0,644 g du produit du stade B et 8 ml de méthanol, puis on dilue 0,565 g de produit obtenu dans 5 ml d'acétate d'éthyle. On refroidit à 0°C et introduit 2,5 ml d'une solution 2,1N d'acide chlorhydrique dans le méthanol et ramène à la température ambiante. On maintient l'agitation à la température ambiante pendant 1 heure. On évapore les solvants, dilue à l'eau, verse sur une solution aqueuse saturée de carbonate de sodium, filtre et concentre. On obtient 0,508 g d'une huile que l'on chromatographie sur silice CH₂Cl₂/MeOH (93-7) puis CH₂Cl₂/MeOH/NH₄OH (93-7-0,5). On concentre les fractions homogènes en CCM, reprend à l'acétate d'éthyle, lave à l'ammoniaque, dilue, extrait à l'acétate d'éthyle, à l'eau, sèche, filtre et concentre. On obtient 66 mg de produit recherché.
RMN CDCl₃ ppm
0,85 (t) : CH₃-CH₂ ; 1,00 (d) - 1,15 (d) - 1,25 (d) 1,31 (d) - 1,40 (d) : les CH₃-CH ; 1,34 et 1,51 : 6 et 12 Me ; 2,26 (s) : N(Me)₂ ; 2,44 (m) : H'₄ ; 2,60 (m) : H₈ ; 2,68 (s) : 6-OMe ; 3,03 (m) : H₄ et H₁₀ ; 3,18 (dd) : H'₂ ; 3,55 (m) : H'₅ ; 3,76 (s) - 3,92 (m) - 4,38 (m) : OCH₂CH₂N ; 3,87 (s) : H₁₁ ; 3,83 (q) : H₂ ; 4,24 (d) : H₅ ; 4,31 (d) : H'₁ ; 4,96 (dd) : H₁₃ ; 4,99 (d) - 5,37 (d) : NCH₂O . 7,36 (d) - 7,54 (d) : H imidazole ; 7,30 (ddd) : H₅ ; 8,08 (dt) : H₄ - 8,45 (dd) : H₆ - 8,95 (ddd) : pyridine.

### EXEMPLE 3 : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[(2-phényléthoxy)méthyl] imino]]-érythromycine

### Stade A : 2'-acétate de 2,3-didéhydro-11,12-didéoxy-3-O-de(2,6-didéoxy-3-C-méthyl-3-O-méthyl-alpha-L-ribo-hexopyranosyl)-6-O-méthyl-12,11-[oxycarbony[[(2-phényléthoxy) méthyl] imino]]-3-O-[[2-(triméthylsilyl) éthoxy] méthyl]-érythromycine

On ajoute à 10°C, 18 mg d'hydrure de sodium à 50 % dans l'huile, dans une solution renfermant 2 ml de DMF et 203 mg du produit obtenu au stade A de l'exemple 1.

On agite pendant 15 minutes. On refroidit à -5°C et ajoute 46 µl de chlorométhylphényl éther en solution dans 0,5 ml de DMF.

On évapore le DMF, reprend à l'acétate d'éthyle, lave à l'eau, sèche, filtre et concentre. On obtient 0,277 g de produit recherché.

### Stade B : 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[(2-phényléthoxy)méthyl] imino]]-érythromycine

On refroidit à +5°C et introduit en une fois 0,227 g de produit obtenu au stade A dans 0,33 ml une solution 0,19N d'acide chlorhydrique dans l'acétate d'éthyle. On amène à la température ambiante et agite pendant 2 heures. On refroidit à 0°C, ajoute de l'eau, amène le pH à 9-10 avec une solution concentrée d'ammoniaque, extrait à l'acétate d'éthyle, lave à l'eau, sèche, filtre et concentre. On obtient 0,201 g de produit que l'on dilue dans 2,5 ml de méthanol. On porte au reflux pendant 2 h 30. On évapore le méthanol et obtient 0,188 mg de produit que l'on chromatographie sur silice en éluant avec le mélange chlorure de méthylène-ésopropanol-ammoniaque (95-5-0,5). On concentre, reprend au chlorure de méthylène, ajoute une goutte d'ammoniaque concentré, agite, sèche, filtre et concentre. On obtient 55 mg de produit que l'on essore et sèche à 80°C. On obtient 36 mg de produit F = 210° ~ 212°C.
RMN CDCl₃ ppm
0,88 (t) : CH₃-CH₂ ; 1,02 (d) - 1,15 (d) - 1,25 (d) - 1,31 (d) - 1,39 (d) : les CH₃-CH ; 1,33 - 1,51 : 6 et 12 Me ; 2,27 (s) : N(Me)₂ ; 2,45 (m) : H'₃ ; 2,62 (m) : H₈ ; 2,70 (s) : 6-OMe ; 2,91 (m) : CH₂Φ ; 3,08 (ql) : H₁₀ ; ~ 3,15 (m) : H₄ ; 3,18 (dd) : H'₂ ; ~ 3,58 (m) : H'₅ ; ~ 3,58 et 3,77 : OCH₂ ; 3,87 (q) : H₂ ; 3,91 (s) : H₁₁ ; 4,25 (d) : H₅ ; 4,32 (d) : H'₁ ; 5,00 (d) - 5,33 (d) : NCH₂O ; 5,05 (dd) : H₁₃ ; 7,13 à 7,30 : phényle, éther (~ 0,3 mole).

En opérant comme indiqué ci-dessus, on a préparé le produit suivant :

### EXEMPLE 4 : 11,12-didéoxy-3-de ((2,6-didéoxy-3-C-méthyl-3-O-méthyl-alpha-L-ribohexopyranosyl) oxy) 6-O-méthyl-3-oxo-12,11-(oxycarbonyl (((2-(triméthylsilyl) éthoxy) méthyl) imino)) érythromycine.

F = 141-143°C ; Rf = 0,38 (AcOEt-TEA 95-5).

### EXEMPLE DE COMPOSITION PHARMACEUTIQUE

On a préparé des composés renfermant :
Produit de l'exemple 2 150 mg
Excipient q.s.p. 1 g
Détail de l'excipient : amidon, talc, stéarate de magnésium

### ETUDE PHARMACOLOGIQUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

| Souches bactériennes à GRAM+ | | |
|---|---|---|
| Produits | Ex. 2 | Ex. 3 |
| Staphylococcus aureus 011UC4 | 0,08 | 0,04 |
| Staphylococcus aureus 011G025I | 0,08 | 0,15 |
| Staphylococcus epidermidis 012GO11I | 0,08 | 0,08 |
| Streptococcus pyogenes groupe A 02A1UC1 | ≤ 0,02 | ≤ 0,02 |
| Streptococcus agalactiae groupe B 02B1HT1 | ≤ 0,02 | ≤ 0,02 |
| Streptococcus faecalis groupe D 02D2UC1 | ≤ 0,02 | ≤ 0,04 |
| Streptococcus faecium groupe D 02D3HT1 | ≤ 0,02 | ≤ 0,02 |
| Streptococcus sp groupe G 02G0GR5 | ≤ 0,02 | ≤ 0,02 |
| Streptococcus mitis 02mitCB1 | ≤ 0,02 | ≤ 0,02 |
| Streptococcus mitis 02mitGR16I | ≤ 0,02 | ≤ 0,02 |
| Streptococcus agalactiae groupe B 02B1SJ1 | 0,08 | 0,04 |
| Streptococcus pneumoniae 032UC1 | 0,02 | ≤ 0,02 |

De plus, le produit de l'exemple 1 a montré une activité intéressante sur les souches bactériennes à gram⊖ suivantes : Haemophilus Influenzae 351HT3, 351CB12, 351CA1 et 351GR6.

## Revendications

1. Les composés de formule (I) : 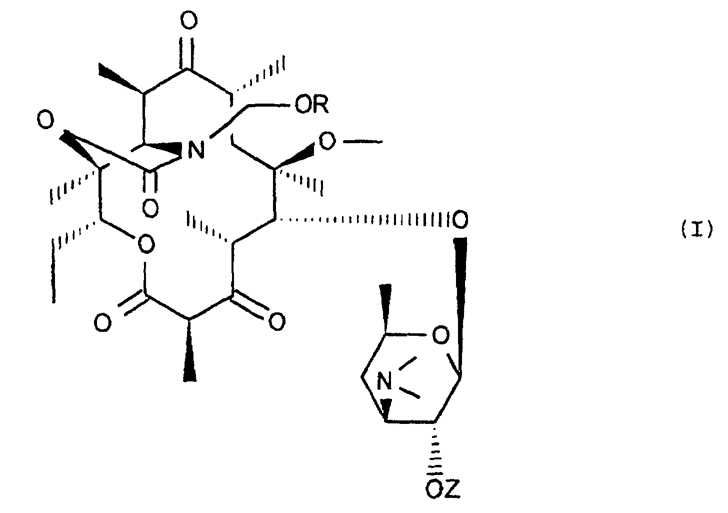 dans lesquels R représente ou bien un radical alkyle, alkényle ou alkynyle, linéaire, ramifié ou cyclique renfermant jusqu'à 18 atomes de carbone, éventuellement substitué par un ou plusieurs substituants choisis parmi les atomes d'halogène, les radicaux O-alkyle, O-alkényle ou O-alkynyle, S-alkyle, S-alkényle ou S-alkynyle, linéaires, ramifiés ou cycliques, renfermant jusqu'à 12 atomes de carbone, les radicaux NO₂, les radicaux C≡N, les radicaux : 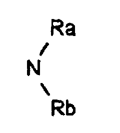 dans lesquels Ra et Rb identiques ou différents représentent un atome d'hydrogène, un radical alkyle linéaire ou ramifié renfermant jusqu'à 4 atomes de carbone, les radicaux : 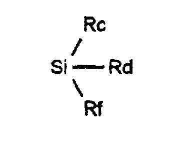 dans lesquels Rc, Rd et Rf identiques ou différents, représentent un radical alkyle, linéaire, ramifié ou cyclique renfermant jusqu'à 4 atomes de carbone, éventuellement substitué par un ou plusieurs atomes d'halogène,
ou bien un radical (CH₂)ₙAr dans lequel n représente un nombre entier allant de 0 à 6 et Ar représente un radical aryle ou hétéroaryle, éventuellement substitué par un ou plusieurs substituants indiqués ci-dessus,
Z représente un atomes d'hydrogène ou le reste d'un radical acyle, renfermant jusqu'à 12 atomes de carbone, ainsi que leurs sels d'addition avec les acides.

2. Les composés de formule (I) tels que définis à la revendication 1, dans lesquels Z représente un atome d'hydrogène.

3. Les composés de formule (I) tels que définis à la revendication 1 ou 2, dans lesquels R représente un radical (CH₂)ₙAr dans lesquels n représente un nombre entier allant de 1 à 4 et Ar représente un radical aryle ou hétéroaryle éventuellement substitué.

4. Les composés de formule (I) définis à la revendication 3 dans lesquels Ar est un radical phényle éventuellement substitué.

5. Les composés de formule (I) définis à la revendication 3 dans lesquels R représente un radical : 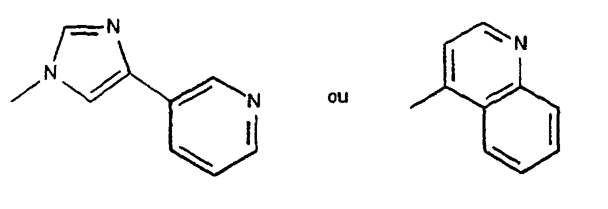 éventuellement substitué.

6. Les composés de formule (I) dont les noms suivent :
- 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[[2-[4-(3-pyridinyl)-1H-imidazol-1-yl] éthoxy] méthyl] imino]]-érythromycine
- 11,12-didéoxy-3-de[(2,6-didéoxy-3-C-méthyl-3-O-méthyl-alpha-L-ribo-hexopyranosyl) oxy]-6-O-méthyl-3-oxo-12,11-[oxycarbonyl[[(2-phényléthoxy)méthyl] imino]]-érythromycine.

7. A titre de médicaments, les composés de formule (I) définis à l'une quelconque des revendications 1 à 6.

8. Les compositions pharmaceutiques renfermant comme principe actif au moins un médicament selon la revendication 7.

9. Procédé de préparation des composés de formule (I), **caractérisé en ce que** l'on soumet un composé de formule (II) : 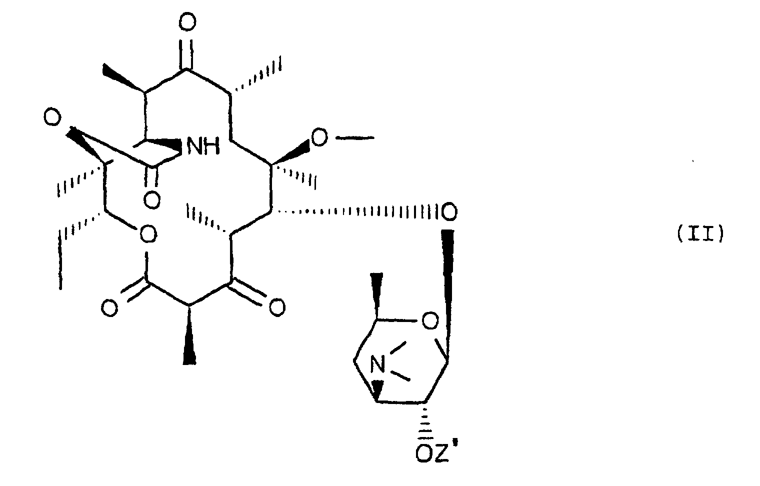 dans lequel Z' représente le reste d'un acide carboxylique renfermant jusqu'à 12 atomes de carbone, à l'action d'un agent de blocage de la fonction cétone en 3 sous forme d'éther ou d'ester d'énol pour obtenir le composé de formule (III) : 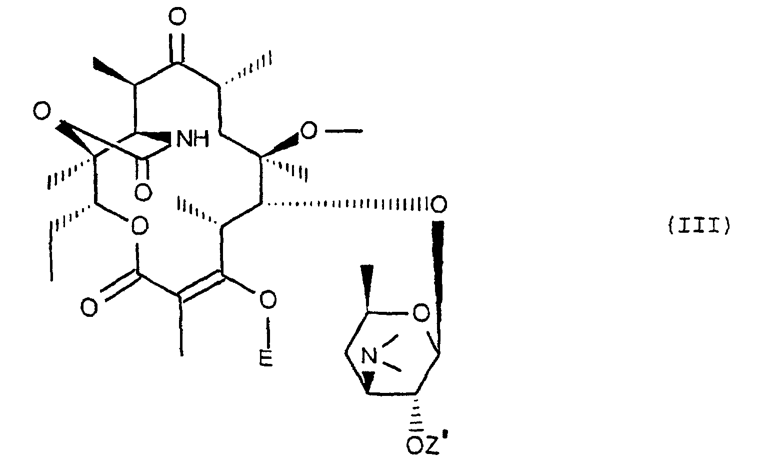 dans lequel E représente le reste d'un éther ou d'un ester d'énol et Z' conserve sa signification précédente, à l'action d'un composé de formule (IV) :
Hal-CH₂OR (IV)
dans laquelle Hal représente un atome d'halogène, pour obtenir le composé de formule (V) correspondant : 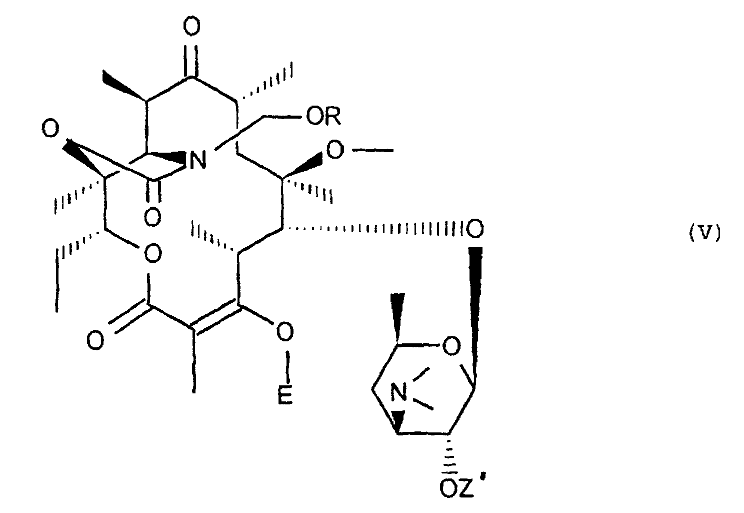 que l'on soumet, si désiré, à l'action d'un agent de libération de la fonction cétone en 3 et/ou à l'action d'un agent de libération de l'hydroxyle en 2', pour obtenir le composé de formule (I) correspondant : 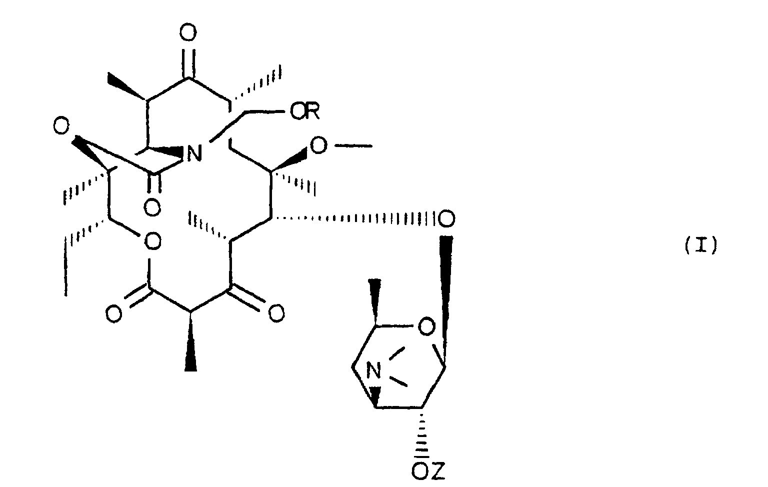 dans lequel Z et R conservent leur signification précédente.

10. Les composés de formules (III) et (V) définis à la revendication 9.

## Patentansprüche

1. Verbindungen der Formel (I) 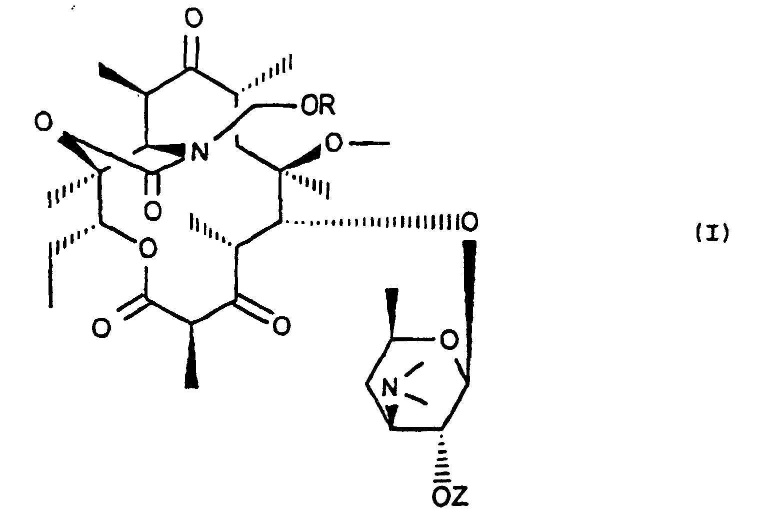 in der
R entweder einen linearen, verzweigten oder cyclischen Rest Alkyl, Alkenyl oder Alkinyl mit bis zu 18 Kohlenstoffatomen darstellt, gegebenenfalls substituiert durch einen oder mehrere Substituenten, ausgewählt unter den Halogenatomen, den linearen, verzweigten oder cyclischen Resten O-Alkyl, O-Alkenyl oder O-Alkinyl, S-Alkyl, S-Alkenyl oder S-Alkinyl mit bis zu 12 Kohlenstoffatomen, den Resten NO₂, den Resten C≡N, den Resten 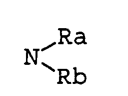 worin Ra und Rb, gleich oder verschieden, ein Wasserstoffatom, einen linearen oder verzweigten Rest Alkyl mit bis zu 4 Kohlenstoffatomen bedeuten, den Resten 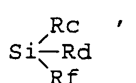 worin Rc, Rd und Rf, gleich oder verschieden, einen linearen, verzweigten oder cyclischen Rest Alkyl mit bis zu 4 Kohlenstoffatomen darstellen, gegebenenfalls substituiert durch ein oder mehrere Halogenatome, oder
einen Rest (CH₂)ₙAr bedeutet, worin n eine ganze Zahl von 0 bis 6 ist und Ar einen Rest Aryl oder Heteroaryl darstellt, gegebenenfalls substituiert durch einen oder mehrere der oben genannten Substituenten,
Z ein Wasserstoffatom oder den Rest eines Acylrestes mit bis zu 12 Kohlenstoffatomen darstellt,
sowie ihre Additionssalze mit Säuren.

2. Verbindungen der Formel (I) wie in Anspruch 1 definiert, worin Z ein Wasserstoffatom darstellt.

3. Verbindungen der Formel (I) wie in Anspruch 1 oder 2 definiert, worin R einen Rest (CH₂)ₙAr darstellt, in dem n eine ganze Zahl von 1 bis 4 ist und Ar einen Rest Aryl oder Heteroaryl bedeutet, gegebenenfalls substituiert.

4. Verbindungen der Formel (I) wie in Anspruch 3 definiert, worin Ar einen Rest Phenyl darstellt, gegebenenfalls substituiert.

5. Verbindungen der Formel (I) wie in Anspruch 3 definiert, worin R einen Rest 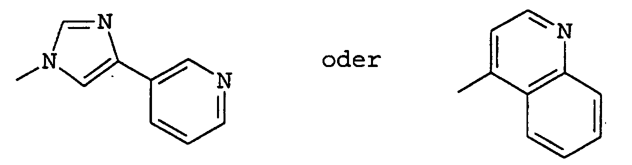 darstellt, gegebenenfalls substituiert.

6. Verbindungen der Formel (I) mit den folgenden Namen:
- 11,12-Dideoxy-3-de-[(2,6-dideoxy-3-C-methyl-3-O-methyl-alpha-Lribohexopyranosyl)-oxy]-6-O-methyl-3-oxo-12,11-{oxycarbonyl-{[[2-(4-(3-pyridinyl)-1H-imidazol-1-yl)-ethoxy]-methyl]-imino}}erythromycin,
- 11,12-Dideoxy-3-de-[(2,6-dideoxy-3-C-methyl-3-O-methyl-alpha-Lribohexopyranosyl)-oxy]-6-O-methyl-3-oxo-12,11-{oxycarbonyl-[[(2-phenylethoxy)-methyl]-imino]}-erythromycin.

7. Als Arzneimittel die Verbindungen der Formel (I) wie in irgendeinem der Ansprüche 1 bis 6 definiert.

8. Pharmazeutische Zusammensetzungen, enthaltend als Wirkstoff mindestens ein Arzneimittel nach Anspruch 7.

9. Verfahren zur Herstellung der Verbindungen der Formel (I), **dadurch gekennzeichnet, daß** man eine Verbindung der Formel (II) 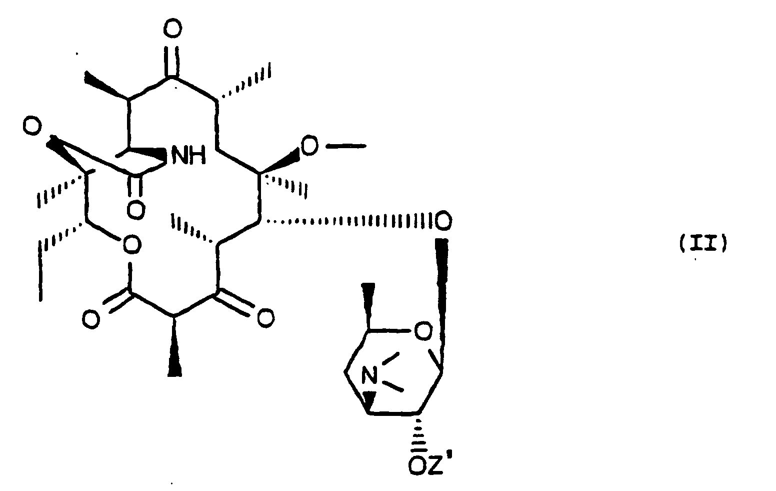 in der Z' den Rest einer Carbonsäure mit bis zu 12 Kohlenstoffatomen darstellt, der Einwirkung eines Mittels zur Blockierung der Keton-Funktion in 3 in Form von Ether oder Enolester unterzieht, um die Verbindung der Formel (III) 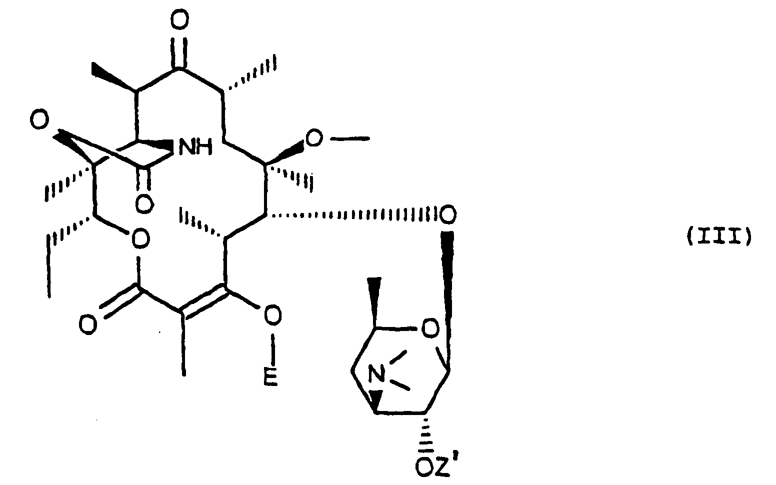 zu erhalten, in der E den Rest eines Ethers oder eines Enolesters darstellt und Z' seine vorstehend genannte Bedeutung beibehält, die man der Einwirkung einer Verbindung der Formel (IV)
Hal-CH₂OR (IV)
unterzieht, in der Hal ein Halogenatom bedeutet, um die entsprechende Verbindung der Formel (V) 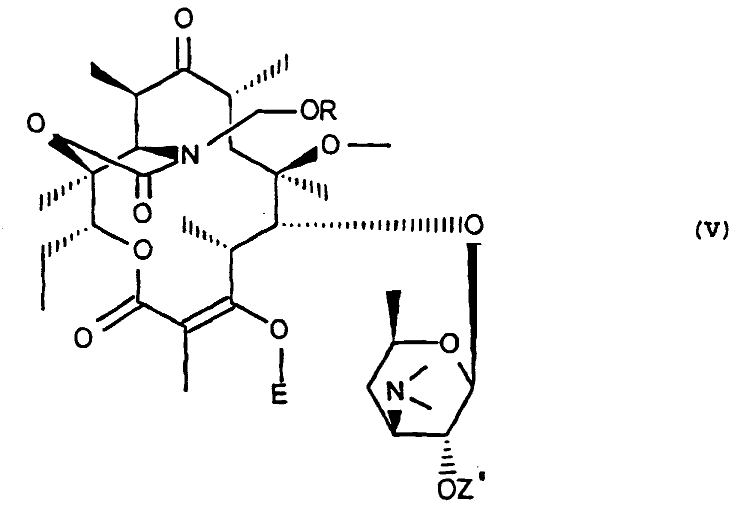 zu erhalten, die man dann, wenn erwünscht, der Einwirkung eines Mittels zur Freisetzung der Ketonfunktion in 3 und/oder der Einwirkung eines Mittels zur Freisetzung des Hydroxyls in 2' unterzieht, um die entsprechende Verbindung der Formel (I) 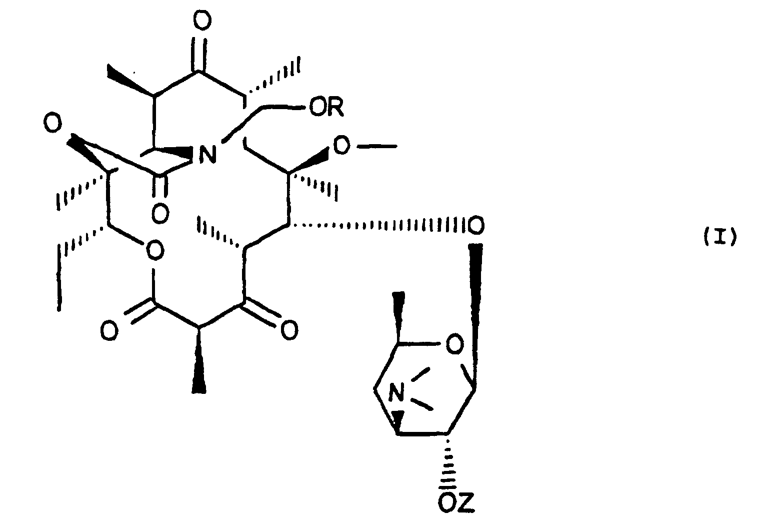 zu erhalten, in der Z und R ihre vorstehend genannte Bedeutung beibehalten.

10. Verbindungen der Formeln (III) und (V) wie in Anspruch 9 definiert.

## Claims

1. The compounds of formula (I): 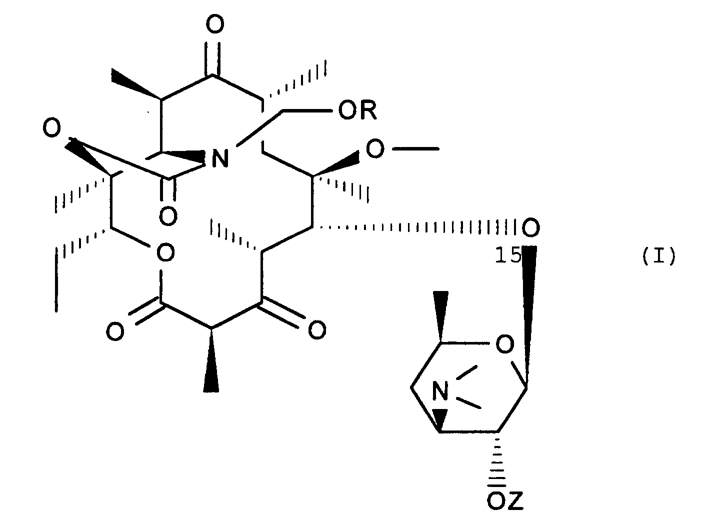 in which R represents or a linear, branched or cyclic alkyl, alkenyl or alkynyl radical containing up to 18 carbon atoms, optionally substituted by one or more substituents chosen from the halogen atoms, the linear, branched or cyclic O-alkyl, O-alkenyl or O-alkynyl, S-alkyl, S-alkenyl or S-alkynyl radicals containing up to 12 carbon atoms, the NO₂ radicals, the C≡N radicals, the 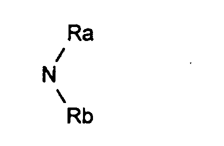 radicals in which Ra and Rb identical or different represent a hydrogen atom, a linear or branched alkyl radical containing up to 4 carbon atoms, the 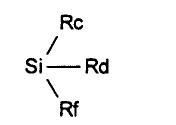 radicals in which Rc, Rd and Rf identical or different, represent a linear, branched or cyclic alkyl radical containing up to 4 carbon atoms, optionally substituted by one or more halogen atoms,
or a (CH₂)ₙAr radical in which n represents an integer between 0 and 6 and Ar represents an aryl or heteroaryl radical, optionally substituted by one or more substituents indicated above,
Z represents a hydrogen atom or the remainder of an acyl radical, containing up to 12 carbon atoms, as well as their addition salts with acids.

2. The compounds of formula (I) as defined in claim 1, in which Z represents a hydrogen atom.

3. The compounds of formula (I) as defined in claim 1 or 2, in which R represents a (CH₂)ₙAr radical in which n represents an integer between 1 and 4 and Ar represents an optionally substituted aryl or heteroaryl radical.

4. The compounds of formula (I) defined in claim 3 in which Ar is an optionally substituted phenyl radical.

5. The compounds of formula (I) defined in claim 3 in which R represents an optionally substituted 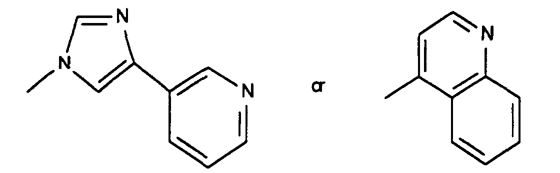 radical.

6. The compounds of formula (I) the names of which follow:
- 11,12-dideoxy-3-de[(2,6-dideoxy-3-C-methyl-3-O-methyl-alpha-L-ribo-hexopyranosyl) oxy]-6-O-methyl-3-oxo-12,11-[oxycarbonyl[[[2-[4-(3-pyridinyl)-1H-imidazol-1-yl] ethoxy] methyl] imino]]-erythromycin
- 11,12-dideoxy-3-de[(2,6-dideoxy-3-C-methyl-3-O-methyl-alpha-L-ribo-hexopyranosyl) oxy]-6-O-methyl-3-oxo-12,11-[oxycarbonyl[[(2-phenylethoxy)methyl] imino]]-erythromycin.

7. As medicaments, the compounds of formula (I) defined in any one of claims 1 to 6.

8. The pharmaceutical compositions containing as active ingredient at least one medicament according to claim 7.

9. Process for the preparation of the compounds of formula (I), **characterized in that** a compound of formula (II): 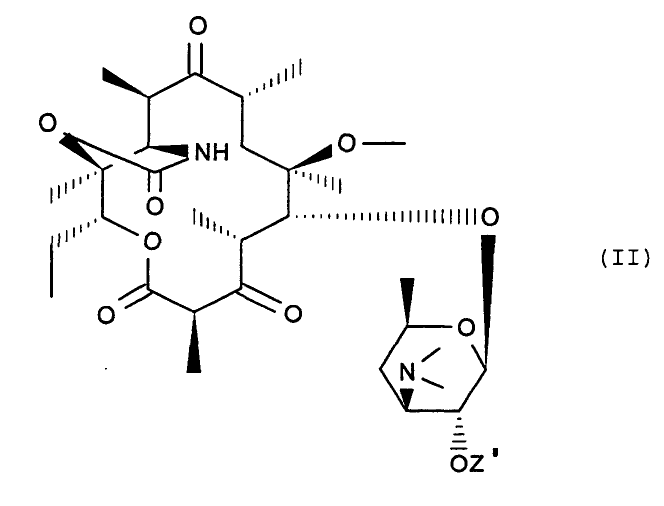 in which Z' represents the remainder of a carboxylic acid containing up to 12 carbon atoms, is subjected to the action of a blocking agent of the ketone function in position 3 in the form of an enol ether or ester in order to obtain the compound of formula (III): 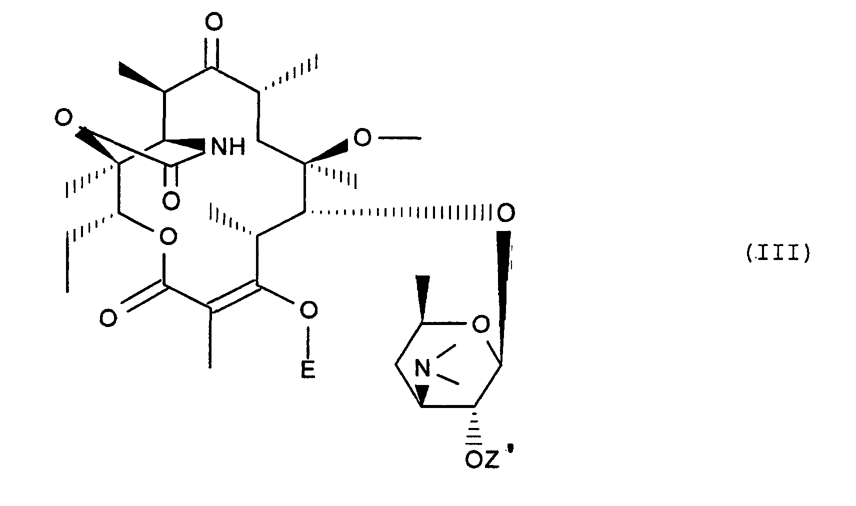 in which E represents the remainder of an enol ether or ester and Z' retains its previous meaning,
is subjected to the action of a compound of formula (IV):
Hal-CH₂OR (IV)
in which Hal represents a halogen atom, in order to obtain the corresponding compound of formula (V): 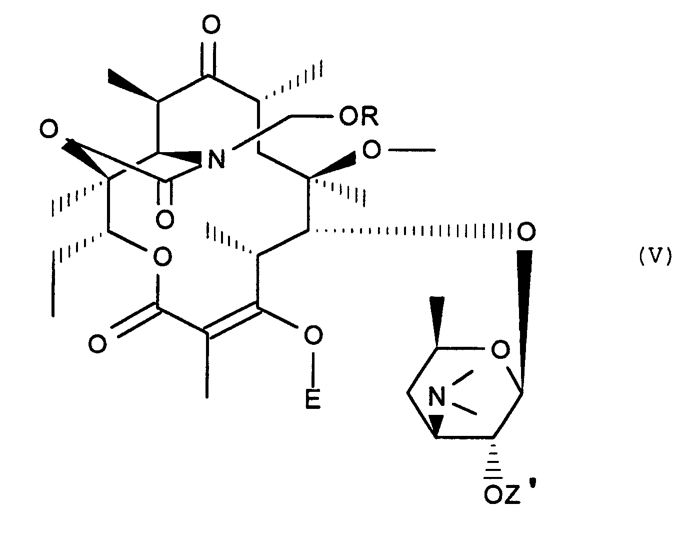 which is subjected, if desired, to the action of an agent which releases the ketone function in position 3 and/or to the action of an agent which releases the hydroxyl in position 2', in order to obtain the corresponding compound of formula (I): 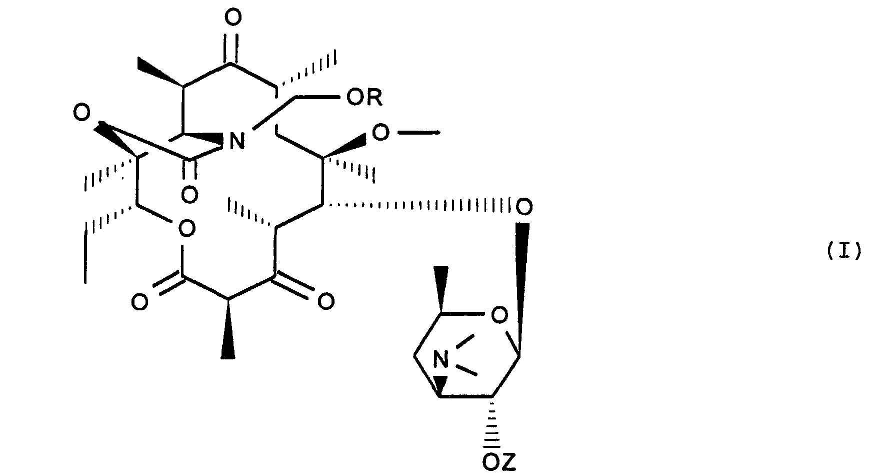 in which Z and R retain their previous meaning.

10. The compounds of formulae (III) and (V) defined in claim 9.
